# EUROPEAN PATENT SPECIFICATION

(11) **EP 0 490 410 B1**
(45) Date of publication and mention of the grant of the patent: **19.06.1996**
(21) Application number: 91121454.2
(22) Date of filing: 13.12.1991
(51) Int. Cl.: C12N 15/12, C12Q 1/68, C07K 14/705

(54) **TXA2 Receptor and gene encoding the same**
TXA2-Rezeptor und dafür kodierendes Gen
Récepteur du TXA2 et gène codant pour celui-ci

(30) Priority: 14.12.1990 JP 410885/90
(43) Date of publication of application: 17.06.1992
(73) Proprietor: SHIONOGI SEIYAKU KABUSHIKI KAISHA, Osaka 541 (JP)
(72) Inventor: Narumiya, Shuh, Nishikyo-ku, Kyoto-shi, Kyoto (JP); Nakanishi, Shigetada, Kyoto-shi, Kyoto (JP)
(74) Representative: VOSSIUS & PARTNER

(56) References cited:
- EP-A- 0 344 024
- NATURE, vol. 349, no. 6310, 14 February 1991, LONDON (GB); M. HIRATA et al., pp. 67-620
- JAPANESE JOURNAL OF PHARMACOLOGY, vol. 55, no. 1, 1991, JP; M. HIRATA et al., p. 167
- JOURNAL BIOLOGICAL CHEMISTRY, vol. 264, no. 28, 05 October 1989, Baltimore, MD (US); F. USHIKUBI et al., pp. 16496-16501
- BIOCHEMICAL & BIOPHYSICAL RESEARCH COMMUNICATIONS, vol. 163, no. 1, 30 August 1989, New York, NY (US); G.W. DORN II, pp. 183-188
- ADVANCES IN PROSTAGLANDIN, THROMBOXANE AND LEUKOTRIENE RESEARCH, vol. 21A, 1990, New York, NY (US); S. NARUMIYA et al., pp. 339-346

## Description

This invention relates to a gene encoding a TXA₂ receptor, a method for producing a TXA₂ receptor with the use of the gene, a TXA₂ receptor obtainable by the method, a host expressing the gene, a probe specifically hybridizing to the gene and a method for detecting the gene with the use of the probe.

Thromboxane (TX) A₂ is a very unstable arachidonate metabolite, yet a potent stimulator of platelet aggregation and a constrictor of vascular and respiratory smooth muscles. It has been implicated as a mediator in diseases such as myocardial infarction, stroke and bronchial asthma. Using S-145, a stable analogue of this compound, the present inventors recently purified the human platelet TXA₂ receptor to apparent homogeneity (Ushikubi, F. et al, J. Biol. Chem. 264, 16496-16501 (1989)).

Bioactive arachidonate metabolites, collectively called eicosanoids, comprise a large family of substances consisting of more than 30 members, and each member of the family acts on a specific receptor to exert a variety of activities. The structure of any member of this family has not yet been revealed. The cloning of one eicosanoid receptor will facilitate elucidation of receptor structures for other members of this family and make way for developing more selective drugs to control these pathophysiologically important mediators.

A relatively large amount of TXA₂ receptor is required to research drugs specifically binding a TXA₂ receptor and to conduct a study of the TXA₂ receptor itself. However, there is no mean for providing a necessary amount of a TXA₂ receptor therefore.

A cell expressing a TXA₂ receptor is indispensable to research for its agonist and antagonist and for elucidation of mechanism of signal-mediation from the receptor.

There is no mean for specifically detecting the expression of a TXA₂ receptor gene in human cells.

Using an oligonucleotide probe corresponding to the partial amino acid sequence of a human platelet TXA₂ receptor, the present inventors have obtained a cDNA clone encoding this receptor from human placenta and a partial clone from cultured human megakaryocytic leukemia cells. The placenta cDNA encodes a protein of 343 amino acids with seven putative transmembrane domains. The protein expressed in COS-7 cells binds drugs with an affinity identical to that of the platelet receptor, and that in *Xenopus* oocytes opens Ca²⁺-activated Cl⁻ channel upon agonist stimulation. Northern blot analysis and the nucleotide sequences of the two clones suggest that an identical species of the TXA₂ receptor is present in platelets and vascular tissues.

The elucidation of the amino acid and nucleotide sequences of the TXA₂ receptor by this invention facilitates elucidation of receptor structures for other members of the TXA₂-belonging family. This invention provides a method for producing the TXA₂ receptor by expressing the gene in a host and the TXA₂ receptor obtainable thereby. This enables one to develop more selective drugs to control pathophysiologically important mediators. This invention further provides a host expressing the gene, which makes it possible to research agonists and antagonists of the TXA₂ receptor and study mechanism of signal-mediation from the receptor. This invention provides a probe specifically hybridizing to the gene, by which the gene can specifically be detected.

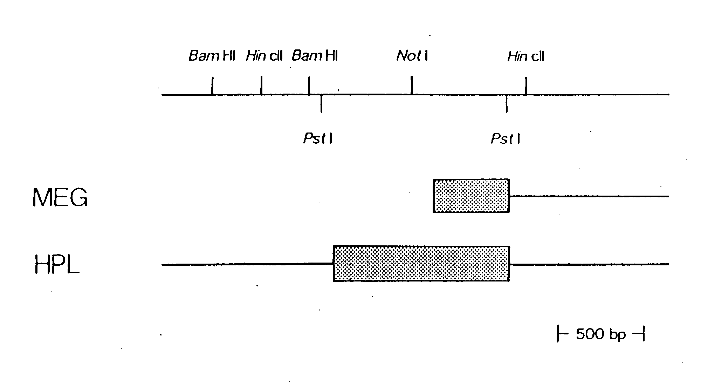

Fig. 1 shows a restriction map of HPL and MEG.

The first object of this invention is to provide a gene encoding a TXA₂ receptor, which desirably has the amino acid sequence of SEQ ID NO: 1. The TXA₂ receptor-encoding gene of this invention includes all genes encoding receptor proteins which show TXA₂ receptor activity and, especially, specifically bind to a compound S-145 as described below, not limited to one gene encoding the amino acid sequence of SEQ ID NO: 1. The gene may contain a sequence useful for its expression, for example, a promoter/operator sequence, an SD sequence, a terminator, a poly-adenylation signal or the like, upstream or downstream of the receptor-coding region. The gene may contain any sequences which do not inhibit its expression. Therefore, this invention comprehends plasmid or viral genes containing the gene and expression vectors for the gene. The gene means DNA and/or RNA, not limited to only one of them.

The second object of this invention is to provide a method for producing a TXA₂ receptor by expressing the above-mentioned gene in a host and a TXA₂ receptor obtainable by the method. The host is preferably eukaryotic. In the method of this invention, not only DNA but also mRNA can be used. The TXA₂ receptor of this invention includes all receptor proteins which show TXA₂ receptor activity and, especially, specifically bind to a compound S-145 as described below, not limited to one protein having the amino acid sequence of SEQ ID NO: 1.

The third object of this invention is to provide a host expressing the above-mentioned gene. The host is preferably eukaryotic and more preferably *Xenopus* oocytes or COS-7 cells. The gene of this invention can be expressed in any conventional host including prokaryotes such as *Escherichia coli* and *Bacillus subtilis* and eukaryotes such as yeast and CHO cells with the use of host/vector systems well known in this field.

The last object of this invention is to provide a probe capable of specifically hybridizing to the above-mentioned gene. The probe includes any probes which specifically hybridize to the gene and is capable of specifically detecting the gene, for example, the HincII fragment (586 bp) of the MEG clone. The probe is not limited to DNA and may be RNA. The probe may preferably be labelled by appropriate labels including radioisotopes such as ³²P. By hybridizing this probe with the gene in a sample according to the usual Northern blot analysis or the like, the gene can specifically be detected.

This invention is further detailed below.

Purified human platelet TXA₂ receptor was subjected to proteolysis, and four partial amino acid sequences were determined. A part of the sequences was used to design a 41-mer oligonucleotide probe. With this probe a cDNA library of MEG-01 cultured human megakaryocytic leukemia cells (Nakajima, M. et al., Biochem. Biophys. Res. Commun. 158, 958-965 (1988)) was screened. One positive clone (MEG) with a 1.4-kilobase (kb) insert was isolated. The nucleotide sequence of MEG revealed that it was a partial clone. Therefore, a 586 base pair (bp) HincII MEG fragment was used as a probe to screen a human placenta cDNA library. Placenta was chosen as a cDNA source because it showed most intense signals on Northern blot analysis. One positive clone, HPL, was isolated that had a 2.9 kb insert containing an entire coding region. When this invention is reproduced, clones encoding a TXA₂ receptor may easily be isolated by screening with the use of probes synthesized according to the DNA sequence of SEQ ID NO: 1.

Fig. 1 and Sequence Listing show the restriction map and nucleotide (2,932 bp) and deduced amino acid sequences derived from HPL. The open reading frame (1,029 bp) codes for a 343-amino-acid protein (relative molecular mass, 37,429). MEG covers the nucleotide sequence from 1,572 to 2,923 of HPL shown in SEQ ID NO: 1 with two substitutions, and the deduced amino acid sequence in the overlapping region is identical. The N-terminal sequence of HPL is identical to that of one of the fragment peptides and there are two putative N-glycosylation sites where no signal was found on peptide analysis. Hydrophobicity analysis of the deduced amino acid sequence revealed seven hydrophobic stretches that could represent transmembrane domains (in SEQ ID NO: 1, I:30-52, II:67-86, III:107-128, IV:150-172, V:194-219, VI:247-270, and VII:290-311).

Comparison of the sequence with those of the rhodopsin-type receptors revealed a significant extent of homology between this and other receptors, particularly in their putative transmembrane regions, suggesting that it belongs to this receptor family. As in the rhodopsin and substance P receptor, there is no Asp in the third transmembrane domain of the TXA₂ receptor which is presumed to serve for binding of the amino group of the ligand in adrenergic receptors. On the other hand, the TXA₂ receptor of this invention has Arg²⁹⁵ which is located at the position analogous to Lys²⁹⁶ of bovine rhodopsin in the seventh transmembrane domain. The latter amino acid residue was assigned for retinal attachment in the rhodopsin molecule. These structural features of the TXA₂ receptor may reflect the acidic nature of the ligand for this receptor.

The third cytoplasmic loop of the TXA₂ receptor consists of less than 30 residues, and the N-terminal end of this loop shows significant homology to rhodopsin. However, the C-terminal end of this loop lacks the basic amino acid cluster which is present in the other receptors. These regions are involved in binding and determining specificity of a coupling G-protein. The G-protein coupling to the TXA₂ receptor is insensitive to pertussis and cholera toxins and remains to be identified.

The short carboxy tail contains six serine and threonine residues and shows some homology to the adrenergic receptors, especially the β₁ receptor. This structure may be related to agonist-induced desensitization of the signalling pathway. Like the β₁ receptors, the TXA₂ receptor undergoes desensitization upon agonist stimulation.

To establish that HPL encodes the TXA₂ receptor, the present inventors subcloned a 2.0 kb fragment of HPL into an eukaryotic expression vector, CDM8 (Seed, B., Nature 329, 840-842 (1987)), and transfected the plasmid into COS-7 cells. Ligand binding was examined with the selective TXA₂ receptor antagonist, [³H]S-145 (Ushikubi, F. et al, Eicosanoids 2, 21-27 (1989)). The membranes of transfected COS cells bound [³H]S-145 in a saturable manner with the dissociation constant, Kd, of 1.2 nM, which is comparable to that observed with membranes from fresh platelets. Specificity of this binding was analysed with several TXA₂ analogues as well as various prostanoids. The TXA₂ agonist, STA₂, and antagonists, S-145 and ONO-3708, competed for the [³H]S-145 binding with an order of potency identical to that observed with the TXA₂ receptor in other cells. Other prostaglandins (PGs) and an inactive TXA₂ metabolite, TXB₂, were at least two orders of magnitude less active in the competition.

The TXA₂ receptor stimulation results in PI turnover and Ca²⁺ mobilization. The present inventors, therefore, expressed the clone in *Xenopus* oocyte where agonist stimulation linked to PI turnover opens endogenous Ca²⁺-dependent Cl⁻ channel. Application of 1 µM STA₂ to the oocytes injected with the *in vitro* transcribed mRNA of the clone evoked a fast inward current of a few hundred nA followed by a slow phase of current of low amplitude. This response was seen at as low as 1 nM STA₂ concentration. PGD₂ and PDF₂ α did not evoke response at 1 µM. Other PGs such as PDE₁, E₂ and I₂ and TXB₂ did not evoke response, either, under these experimental conditions. Thus, the protein encoded by HPL possesses the ligand binding properties characteristics of the TXA₂ receptor and evokes a response expected for this receptor.

Northern blot analysis of the TXA₂ receptor mRNA expression in various tissues is of interest because of a controversy over the presence of the receptor subtypes. As extensive analysis using poly(A)⁺ RNA from various rat tissues yielded little signals probably due to the species difference of the receptor, the present inventors performed the analysis only on poly(A)⁺RNA from human placenta and lung and cultured MEG-01 cells. Placenta and lung are representative tissues rich in the TXA₂ receptor. A major hybridization band was observed at 2.8 kb in all three lanes under stringent conditions, which was most intense with the placenta RNA. In this lane, a minor band was present at 3.5 kb. This band became apparent also in the lane of the MEG-01 poly(A)⁺RNA on extended exposure. These results together with the above findings that MEG and HPL encode the identical amino acid sequence suggest that the same species of mRNA are expressed in platelet precursor cells and vascular rich tissues.

Bioactive arachidonate metabolites, collectively called eicosanoids, comprise a large family of substances consisting of more than 30 members, and each member of the family acts on the specific receptor to exert a variety of activities. This cloning of an eicosanoid receptor will facilitate elucidation of receptor structures for other members of this family and make way for developing more selective drugs to control these pathophysiologically important mediators.

### Example

Human platelet TXA₂ receptor was purified to apparent homogeniety (Ushikubi, F. et al, J. Biol. Chem. 264, 16496-16501 (1989)) and subjected to proteolysis with cyanogen bromide, lysyl endopeptidase and trypsin. Fragment peptides were isolated by reverse phased HPLC and sequenced by automated pulse-liquid phase protein sequencer. Four partial amino acid sequences were obtained. A part of these sequences (amino acid sequence from 296 to 309 of SEQ ID NO: 1) was used to design a 41-mer oligonucleotide probe. MEG-01 cDNA library was screened with this probe radiolabelled at the 5'-end. One positive clone, MEG, was isolated. The sequence of a 1.4 kb insert in this clone revealed an open reading frame which contained two of the partial amino acid sequences of the purified protein. A 586 bp fragment of MEG was then used to screen a placenta cDNA library (T. Maniatis et al, Molecular Cloning (1989), Cold Spring Harbor Laboratory Press, Cold Spring Harbor, NY). One positive clone, HPL, was obtained and analysed. DNA sequencing was carried out on double stranded templates using dideoxy chain termination method.

Fig. 1 shows a restriction map of TXA₂ receptor clones from human placenta (HPL) and cultured human megakaryocytic leukemia cells (MEG). Stippled boxes indicate coding region.

SEQ ID NO: 1 in Sequence Listing shows nucleotide and deduced amino acid sequences of HPL. The most 5' ATG triplet found in frame with all of the partial amino acid sequences of the purified protein was assigned as the site of translation initiation. There are 18 other ATGs in 5'-noncoding region, all of which terminate after short stretch of reading frames. The putative poly-adenylation signal is from 2,908 to 2,913 of SEQ ID NO: 1. Sequences obtained from proteolytic peptides are those of 1-131-141, 277-284 and 289-322 of amino acid sequence shown in SEQ ID NO: 1. The putative N-glycosylation sites are two Asn residues at positions 4 and 16 of the amino acid sequence of SEQ ID NO: 1. MEG covers the nucleotide sequence from 1,572 to 2,923 of HPL shown in SEQ ID NO: 1 with two substitutions; both T→C at the positions of 1,786 and 1,915 of HPL shown in SEQ ID NO: 1. These substitutions do not alter the amino acids encoded by these codons.

### Expression

HPL was digested with BamHI to remove most of the 5'-untranslated region, and its 2.0 kb fragment was inserted between the HindIII-XbaI site of eukaryotic expression vector CDM8 (Seed, B., Nature 239, 840-842 (1987)). A modified DEAE-dextran method (Ausbel, F. M. et al, Current Protocols in Molecular Biology. 9.2.5 (Wiley, New York, 1989)) was used for the transfection of COS-7 cells. At 60 h after the transfection, cells were collected and membranes were prepared by homogenizing the cells according to the method of Ausbel, F. M. et al., as described above in the presence of 1 mM benzamidine and 0.3 mM PMSF. The membranes were finally suspended in 20 mM Na-Mes, pH 6.4, and 5 mM EDTA, and binding experiments with [³H]S-145 (24 Ci/mmol) were carried out as described in Ushikubi, F. et al., Eicosanoids 2, 21-27 (1989). Competition experiments were carried out with 1.25 nM [³H]S-145 and various concentrations of ligands.

The membranes of transfected COS cells bound [³H]S-145 in a saturable manner with the dissociation constant, Kd, of 1.2 nM, which is comparable to that observed with membranes from fresh platelets. The estimated maximal binding, Bmax, was 2.2 pmol/mg protein.

The TXA₂ agonist, STA₂, and antagonists, S-145 and ONO-3708, competed for the [³H]S-145 binding with an order of potency identical to that observed with the TXA₂ receptor in other cells. Other prostaglandins (PGs) and an inactive TXA₂ metabolite, TXB₂, were at least two orders of magnitude less active in the competition.

### Response Reaction

The mRNA synthesized *in vitro* from the 2.0 kb BamHI subclone of HPL was injected into *Xenopus* oocytes and electrophysiological responses were measured after 48 hours. A TXA₂ agonist, STA₂, and other PGs dissolved in ethanol at 100 µM were added to the bath at 1 µM concentration, and responses of oocytes were recorded by the two-micropipette voltage clamp method as described in Masu, Y. et al., Nature 329, 836-838 (1987).

Application of 1 µM STA₂ evoked a fast inward current of a few hundred nA followed by a slow phase of current of low amplitude. This response was seen at as low as 1 nM STA₂ concentration. PGD₂ and PGF₂ α did not evoke response at 1 µM. Other PGs such as PGE₁, E₂ and I₂ and TXB₂ did not evoke response, either, under these experimental conditions. Thus, the protein encoded by HPL possesses the ligand binding properties characteristics of the TXA₂ receptor and evokes a response expected for this receptor.

### Northern Blot Analysis

Total RNA was prepared from each of cultured MEG-01 cells and human placenta and lung tissues using guanidinium thiocyanate-cesium chloride method, and poly(A)⁺ RNA was isolated using a oligo(dT) cellulose column. Twenty µg of each poly(A)⁺ RNA were subjected to formaldehyde agarose gel electrophoresis, transferred to Biodyne membrane and UV-cross-linked. The filter was prehybridised in 5 × SSC buffer, 5 × Denhardt's solution, SO mM sodium phosphate, pH 6.5, 200 µg/ml heat denatured salmon testis DNA, 50 % formamide and 0.1 % SDS at 42°C for 4 h, and hybridised in the same solution at 42°C overnight with a 586 bp HincII fragment of MEG clone labelled with ³²P-dCTP by random-priming (4 × 10⁶ c.p.m.ml⁻¹). The filter was washed twice in 0.1 × SSC, 0.1 % SDS at 65°C for 1 h and exposed to an X-ray film for 12 days in the presence of an intensifying screen at -80°C.

The present inventors performed the analysis only on poly(A)⁺RNA from human placenta and lung and cultured MEG-01 cells. Placenta and lung are representative tissues rich in the TXA₂ receptor. A major hybridization band was observed at 2.8 kb in all three lanes under stringent conditions,which was most intense with the placenta RNA. In this lane, a minor band was present at 3.5 kb. This band became apparent also in the lane of the MEG-01 poly(A)⁺RNA on extended exposure. These results together with the above findings that MEG and HPL encode the identical amino acid sequence suggest that the same species of mRNA are expressed in platelet precursor cells and vascular rich tissues.

### Sequence Listing

SEQ ID NO: 1
SEQUENCE LENGTH: 2,932
SEQUENCE TYPE: nucleic acid
STRANDEDNESS: double
TOPOLOGY: linear
MOLECULAR TYPE: cDNA to mRNA
HYPOTHETICAL: No
ANTI-SENSE: No
ORIGINAL SOURCE
   ORGANISM: human
   TISSUE TYPE: placenta
   CLONE: HPL
FEATURE
   KEY: polyA signal
   LOCATION: 2908..2913
   IDENTIFICATION METHOD: S

SEQUENCE DESCRIPTION

GTAATGCAGA GATAATAAAA CTTCTTAGGT CCATAGGTCT TATAATAATT TAATAACCTA 60

AACATGGTAT ACAAATTCCT CCAAACCCAA TAACATAATT ATAGTTTCAA AAAGTTCCCC 120

AAACTTTCAA GTTAGATTTT ATTGCTTTGA TGAGTGGCTT TAAATATGAA AAGTCTTGCC 180

TGTGAAGGGC AATCCTTTTC CCGTGGACTG GGATCTATAG AAATACAGAA ATGTGCCCAG 240

GGGTTCATCT CCCTAATAAC CATCATTCAC ATTTCTCAAC CTCCCTAATA ACCAGCCACC 300

ATGTGAGAAG GATCCACAGT TACTGTTTAT GACTATAATT AACTAGTACC TGGGACTGGT 360

CAGTGGAGTT GGTTGCAACC TGATGCTAAG GATGTCAAAG TTGTCTCGGC CTCTGTTCCC 420

AGCCAGTAAG TAATTCCCTG GCCTCGGGCC ATACCCCCTA ATCTTGGTCA GCTGATTATG 480

ACAGGCAGAC AGCACAGTAA ATAACACTAT ATATTAAGAA AACCCAAAGC ATATGTATCA 540

ATGGTATATA CCCAACAGCA TCCTAGGAAT GGAGAGTCTG TAGCAAGGGC CTCCAATGTG 600

AAGGTCAACA CAGTCACTGT GATGCGTGTA TTTCCATTTT GTAAAGCATG ATCTCTGGTG 660

GTCATTTTTA TCTTCCTAAC TTATTGGAAA AGTCTCCTGT TTTGGGGGCC CGCCCCTGGT 720

CACAGCCAGA CTGACTCAGT TTCCCTGGGA GGTCCCGCTC GAGCCCGTCC TTCCCCTCCC 780

TCTGCCCGCC CCCAGCCCTC GCCCCACCCT CGGCGCCCGC ACATCTGCCT GCTCAGCTCC 840

AGACGGCGCC CGGACCCCCG GGCGCGGGAT CCAGCCAGGT GGGAGCCCCG CAGATGAGGT 900

CTCTGAAGGT GTGCCTGAAC CAGTGCCAGC CTGCCCTGTC TGCAGCATCG GCCTGATGGG 960

GTGGTGACTG ATCCCTCAGG GCTCCGGAGC C ATG TGG CCC AAC GGC AGT TCC 1012



1               5

CTG GGG CCC TGT TTC CGG CCC ACA AAC ATT ACC CTG GAG GAG AGA CGG 1060



        10                  15                  20

CTG ATC GCC TCG CCC TGG TTC GCC GCC TCC TTC TGC GTG GTG GGC CTG 1108



    25                  30                  35

GCC TCC AAC CTG CTG GCC CTG AGC GTG CTG GCG GGC GCG CGG CAG GGG 1156



40                  45                  50                  55

GGT TCG CAC ACG CGC TCC TCC TTC CTC ACC TTC CTC TGC GGC CTC GTC 1204



                60                  65                  70

CTC ACC GAC TTC CTG GGG CTG CTG GTG ACC GGT ACC ATC GTG GTG TCC 1252



            75                  80                  85

CAG CAC GCC GCG CTC TTC GAG TGG CAC GCC GTG GAC CCT GGC TGC CGT 1300



        90                  95                  100

CTC TGT CGC TTC ATG GGC GTC GTC ATG ATC TTC TTC GGC CTG TCC CCG 1348



    105                 110                 115
CTG CTG CTG GGG GCC GCC ATG GCC TCA GAG CGC TAC CTG GGT ATC ACC 1396

120                 125                 130                 135
CGG CCC TTC TCG CGC CCG GCG GTC GCC TCG CAG CGC CGC GCC TGG GCC 1444

                140                 145                 150
ACC GTG GGG CTG GTG TGG GCG GCC GCG CTG GCG CTG GGC CTG CTG CCC 1492

            155                 160                 165
CTG CTG GGC GTG GGT CGC TAC ACC GTG CAA TAC CCG GGG TCC TGG TGC 1540

        170                 175                 180
TTC CTG ACG CTG GGC GCC GAG TCC GGG GAC GTG GCC TTC GGG CTG CTC 1588

    185                 190                 195
TTC TCC ATG CTG GGC GGC CTC TCG GTC GGG CTG TCC TTC CTG CTG AAC 1636

200                 205                 210                 215
ACG GTC AGC GTG GCC ACC CTG TGC CAC GTC TAC CAC GGG CAG GAG GCG 1684

                220                 225                 230
GCC CAG CAG CGT CCC CGG GAC TCC GAG GTG GAG ATG ATG GCT CAG CTC 1732

            235                 240                 245
CTG GGG ATC ATG GTG GTG GCC AGC GTG TGT TGG CTG CCC CTT CTG GTC 1780

        250                 255                 260
TTC ATT GCC CAG ACA GTG CTG CGA AAC CCG CCT GCC ATG AGC CCC GCC 1828


    265                 270                 275
GGG CAG CTG TCC CGC ACC ACG GAG AAG GAG CTG CTC ATC TAC TTG CGC 1876

280                 285                 290                 295
GTG GCC ACC TGG AAC CAG ATC CTG GAC CCC TGG GTG TAT ATC CTG TTC 1924

                300                 305                 310
CGC CGC GCC GTG CTC CGG CGT CTC CAG CCT CGC CTC AGC ACC CGG CCC 1972

            315                 320                 325
AGG TCG CTG TCC CTC CAG CCC CAG CTC ACG CAG CGC TCC GGG CTG CAG 2020

        330                 335                 340
TAGGAAGTGG ACAGAGCGCC CCTCCCGCGC CTTTCCGCGG AGCCCTTGGC CCCTCGGACA 2080
GCCCATCTGC CTGTTCTGAG GATTCAGGGG CTGGGGGTGC TGGATGGACA GTGGGCATCA 2140
GCAGCAGGGT TTTGGGTTGA CCCCAATCCA ACCCGGGGAC CCCCAACTCC TCCCTGATCC 2200
TTTTACCAAG CACTCTCCCT TCCTCGGCCC CTTTTTCCCA TCCAGAGCTC CCACCCCTTC 2260
TCTGCGTCCC TCCCAACCCC AGGAAGGGCA TGCAGACATT GGAAGAGGGT CTTGCATTGC 2320
TATTTTTTTT TTTAGACGGA GTCTTGCTCT GTCCCCCAGG CTGGAGTGCA GTGGCGCAAT 2380
CTCAGCTCAC TGCAACCTCC ACCTCCCGGG TTCAAGCGAT TCTCCTGCCT CAGCCTCCTG 2440
AGTAGCTGGG ACTATAGGCG CGCGCCACCA CGCCCGGCTA ATTTTTGTAT TTTTAGTAGA 2500
GACGGGGTTT CACCGTGTTG GCCAGGCTGG TCTTGAACTC CTGACCTCAG GTGATTCACC 2560
AGCCTCAGCC TCCCAAAGTG CTGGGATCAC AGGCATGAAC CACCACACCT GGCCATTTTT 2620
TTTTTTTTTT TAGACGGAGT CTCACTCTGT GGCCCAGCCT GGAGTACAGT GGCACGATCT 2680
CGGCTCACTG CAACCTCCGC CTCCCGGGTT CAAGCGATTC TCGTGCCTCA GCCTCCCGAG 2740
CAGCTGGGAT TACAGGCGTA AGCCACTGCG CCCGGCCTTG CATGCTCTTT GACCCTGAAT 2800
TTGACCTACT TGCTGGGGTA CAGTTGCTTC CTTTTGAACC TCCAACAGGG AAGGCTCTGT 2860
CCAGAAAGGA TTGAATGTGA AACGGGGGCA CCCCCTTTTC TTGCCAAAAT ATATCTCTGC 2920
CTTTGGTTTT AT 2932

## Claims

1. An isolated gene encoding a thromboxane A₂ (TXA₂) receptor wherein said TXA₂ receptor contains the amino acid sequence of SEQ ID NO: 1.

2. A method for producing a thromboxane A₂ (TXA₂) receptor which comprises expressing said gene of claim 1 in a host.

3. The method of claim 2 wherein said host is eukaryotic.

4. A host expressing said gene of claim 1.

5. The host of claim 4 which is eukaryotic.

## Patentansprüche

1. Isoliertes Gen, das einen Thromboxan A₂ (TXA₂)-Rezeptor codiert, wobei der TXA₂-Rezeptor die in SEQ ID NO: 1 angegebene Aminosäuresequenz enthält.

2. Verfahren zur Herstellung eines Thromboxan A₂ (TXA₂)-Rezeptors, das die Expression des Gens nach Anspruch 1 in einem Wirt umfaßt.

3. Verfahren nach Anspruch 2, wobei der Wirt ein eukaryontischer Wirt ist.

4. Wirt, der das Gen nach Anspruch 1 exprimiert.

5. Wirt nach Anspruch 4, der ein eukaryontischer Wirt ist.

## Revendications

1. Gène isolé codant un récepteur de thromboxane A₂ (TXA₂), dans lequel ledit récepteur de TXA₂ contient la séquence d'acides aminés de SEQ ID NO:1.

2. Procédé de production d'un récepteur de thromboxane A₂ (TXA₂), comprenant une expression du gène suivant la revendication 1 dans un hôte.

3. Procédé suivant la revendication 2, caractérisé en ce que l'hôte est eucaryote.

4. Hôte exprimant le gène suivant la revendication 1.

5. Hôte suivant la revendication 4, caractérisé en ce qu'il est eucaryote.
